# EUROPÄISCHE PATENTSCHRIFT

(11) **EP 2 339 981 B1**
(45) Veröffentlichungstag und Bekanntmachung des Hinweises auf die Patenterteilung: **04.06.2014**
(21) Anmeldenummer: 09752096.9
(22) Anmeldetag: 10.09.2009
(51) Int. Cl.: A61B 18/22, A61C 1/00

(54) **LASER-HANDSTÜCK SOWIE VERFAHREN ZU SEINER HERSTELLUNG**
LASER HANDPIECE AND METHOD FOR THE PRODUCTION THEREOF
PIÈCE À MAIN LASER ET PROCÉDÉ DE FABRICATION

(30) Priorität: 11.09.2008 DE 102008046825
(43) Veröffentlichungstag der Anmeldung: 06.07.2011
(73) Patentinhaber: Starmedtec GmbH, 82319 Starnberg (DE)
(72) Erfinder: FALKENSTEIN, Werner, 82319 Starnberg (DE); WEIDEMANN, Gregor, 81479 München (DE)
(74) Vertreter: Feldkamp, Rainer
(86) Internationale Anmeldenummer: PCT/EP2009/006583
(87) Internationale Veröffentlichungsnummer: WO 2010/028833

(56) Entgegenhaltungen:
- DE-A1- 4 239 829
- DE-U1- 9 010 419
- DE-U1- 20 003 349
- US-A- 5 228 852
- US-A- 5 454 807
- US-A- 5 951 544

## Beschreibung

Die Erfindung bezieht sich auf ein Laser-Handstück der im Oberbegriff des Anspruchs 1 genannten Art sowie ein Verfahren zur Herstellung eines Laser-Handstückes.

Derartige Handstücke werden zur Führung des freien Endes einer Lichtleitfaser verwendet, deren anderes Ende mit einer Laser-Lichtquelle verbunden ist.

Die hierbei verwendeten Lichtleitfasern weisen einen zur Lichtleitung dienenden Kern mit einem Glasmantel auf, der einen von dem Kern abweichenden Brechungsindex aufweist, um das Licht in dem Kern zu führen. Die Einheit aus Kern und Glasmantel wird nachfolgend als Lichtleitfaser bezeichnet. Über dem Glasmantel ist eine zumeist aus Kunststoff bestehende Ummantelung vorgesehen, die den Kern und dessen Glasmantel schützt.

Für externe Laseranwendungen, aber auch bei offen-chirurgischen wie auch laparoskopischen Anwendungen wird für einen präzisen Schneideinsatz ein kurzer Längenabschnitt der Ummantelung von dem freien Ende der dünnen Lichtleitfaser, die einen Außendurchmesser einschließlich der Ummantelung im Bereich von beispielsweise 0,45-0,9 mm aufweist, entfernt, und die Lichtleitfaser wird in ein Führungsstück einer Faserführungshilfe in Form eines Handstückes eingeführt, das ergonomisch an die menschliche Hand angepasst ist.

Bekannte Ausführungen derartiger Handstücke sind ähnlich wie ein Kugelschreiber ausgebildet, bei dem die ummantelte Lichtleitfaser von hinten durch das Handstück geführt wird, das im Inneren über einen Klemmmechanismus verfügt, um die Lichtleitfaser in Längsrichtung festzulegen, wobei das von der Ummantelung befreite freie Ende der Lichtleitfaser aus dem Führungsstück heraus vorspringt.

Ein derartiges Handstück ist beispielsweise aus der US 5 228 852 bekannt. Dieses bekannte Handstück besteht aus einem lang gestreckten Schaft, der an seinem einen Ende ein in den Schaft einsteckbares und an diesem festzulegendes Führungsstück und an seinem anderen Ende eine Klemmvorrichtung für die mit einer Ummantelung versehene Lichtleitfaser aufweist. Ein derartiges Handstück ist nicht nur in der Herstellung aufwändig, sondern auch nur schwer zu autoklavieren, da es zu diesem Zweck zerlegt werden muss.

Der Erfindung liegt die Aufgabe zugrunde, ein Handstück der eingangs genannten Art sowie ein Verfahren zur Herstellung eines Handstückes zu schaffen, das ein Klemmen und präzises Führen der Lichtleitfaser ermöglicht, sicher autoklavierbar ist und eine einfache und kostengünstige Herstellung aufweist.

Diese Aufgabe wird ausgehend von einem Laser-Handstück der eingangs genannten Art erfindungsgemäß dadurch gelöst, dass der Schaft des Laser-Handstücks zusammen mit dem Führungsstück einstückig aus einem Metallrohr hergestellt ist, dessen Innendurchmesser größer als der Außendurchmesser der ummantelten Lichtleitfaser ist wobei das Metallrohr zur Bildung des Führungsstücks an dem ersten Ende durch spanlose Verformung konusförmig derart verformt ist, dass der Innendurchmesser des Führungsstücks im Wesentlichen auf den Außendurchmesser der Lichtleitfaser verringert ist.

Gemäß einer Ausgestaltung der Erfindung ist der Innnendurchmesser des Führungsstücks lediglich an seinem freien Ende im Wesentlichen auf den Außendurchmesser der Lichtleitfaser verringert.

Gemäß einer bevorzugten Ausgestaltung der Erfindung ist das zweite Ende des Schaftes mit einem Außengewinde versehen, auf das eine die Klemmvorrichtung bildende Überwurfmutter aufschraubbar ist, die an ihrem verschlossenen Ende eine Durchgangsbohrung für den Durchgang der ummantelten Lichtleitfaser aufweist, wobei im Inneren der Überwurfmutter eine elastische Scheibe mit einer Durchgangsbohrung für die ummantelte Lichtleitfaser angeordnet ist, die beim Aufschrauben der Überwurfmutter auf das zweite Ende des Schaftes verformt und gegen die Ummantelung der Lichtleitfaser angepresst wird.

Gemäß einer bevorzugten Ausgestaltung der Erfindung ist das zweite Ende des Schaftes mit einem Luer-Lock-Adapter verbunden, durch den die Klemmung der Ummantelung der Lichtleitfaser erfolgt.

Gemäß einer weiteren Ausgestaltung der Erfindung ist das zweite Ende des Schaftes mit einem Touhy-Borst-Adapter verbunden, durch den die Klemmung der Ummantelung der Lichtleitfaser erfolgt.

Auf diese Weise können handelsübliche, in der Medizin verwendete Verbindungselemente zusätzlich zu dem einstückigen Handstück verwendet werden.

Der Innendurchmesser des Führungsstücks kann gegebenenfalls nachbearbeitet werden, um sehr genau auf den Durchmesser der jeweiligen Lichtleitfaser abgestimmt zu werden.

Dieses Führungsstück kann gegebenenfalls gebogen sein, wenn dies für die Anwendung des Handstückes bequemer ist, um die eingeführte Lichtleitfaser an weniger leicht zugängliche Stellen bewegen zu können.

Gemäß einer anderen Ausgestaltung der Erfindung ist das Metallrohr aus leicht verformbaren Metall hergestellt.

In diesem Fall kann das Metallrohr beispielsweise aus Neusilber bestehen so dass das Führungsstück ohne Weiteres von Hand abbiegbar ist.

Bei einem Verfahren zur Herstellung eines Laser-Handstücks, das einen hohlen lang gestreckten Schaft, ein verjüngtes Führungsstück an einem ersten Ende und eine Klemmvorrichtung an dem dem ersten Ende entgegengesetzten zweiten Ende aufweist, wird der Schaft des Laser-Handstücks zusammen mit dem Führungsstück einstückig aus einem mit einer Bohrung versehenen Metallrohr, dessen Innendurchmesser größer als der Außendurchmesser einer durch das Laser-Handstück zu führenden, ummantelten Lichtleitfaser ist, hergestellt, wobei das Führungsstück mittels einer spanlosen Verformung konusförmig derart verformt wird, dass der Innendurchmesser des Führungsstücks auf den Außendurchmesser der an ihrem freien Ende abgemantelten und mit ihrem freien Ende aus dem Führungsstück vorspringenden Lichtleitfaser verringert wird, so dass sich eine präzise Führung der Lichtleitfaser in dem Führungsstück ergibt.

Das abgemantelte freie Ende der Lichtleitfaser kann hierbei entweder ein von einer äußeren Kunststoffummantelung befreites Ende oder ein Ende sein, das bis auf den Kern abgemantelt ist.

Gemäß einer Ausgestaltung der Erfindung ist das Metallrohr ein Edelstahlrohr.

Die Erfindung wird im Folgenden anhand von in der Zeichnung dargestellten Ausführungsbeispielen noch näher erläutert.

In der Zeichnung zeigen:
Figur 1 eine vereinfachte Seitenansicht des Handstückes;
Figur 2 eine Längsschnittsansicht des Handstücks nach Figur 1;
Figur 3 eine vergrößerte Ansicht der in Figur 2 mit dem Kreis B umgebenen Einzelheit einer Ausführungsform des hinteren Endes des Handstückes;
Figur 4 eine perspektivische Ansicht des Handstückes nach den Figuren 1 bis 3;
Figur 5 eine Seiten ansicht einer weiteren Ausführungsform des Handstückes;
Figur 6 eine perspektivische Ansicht der weiteren Ausführungsform des Handstückes.

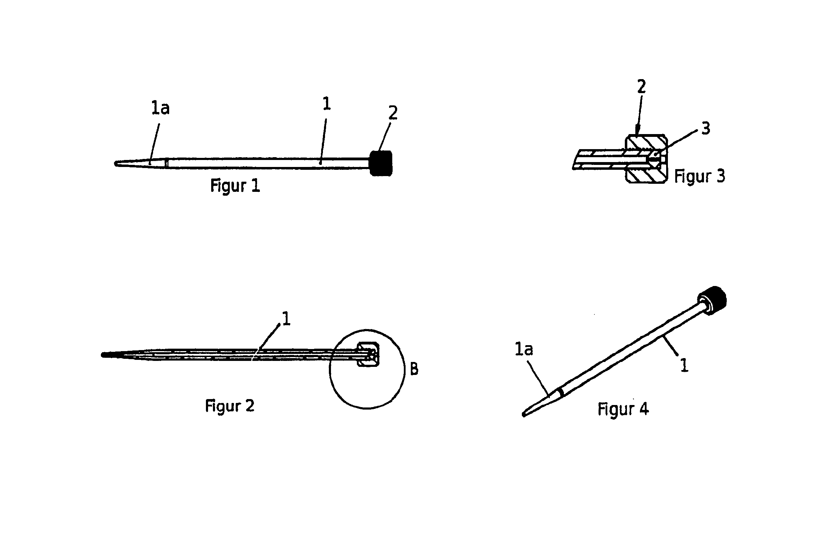

In Figur 1 ist eine Seitenansicht einer Ausführungsform eines Laser-Handstückes 1 gezeigt, das an seinem einen freien Ende ein Führungsstück 1 a aufweist.

Dieses Handstück 1 weist einen hohlen Schaft auf, dessen Innendurchmesser größer als der Außendurchmesser einer ummantelten Lichtleitfaser (nicht gezeigt) ist, die vor dem Einführen in das Handstück 1 an seinem freien Ende abgemantelt wird, um den Kern der Lichtleitfaser und dessen Glasmantel freizulegen, der dann am freien Ende aus dem Führungsstück 1 a vorspringt.

Das Führungsstück 1a wird ausgehend von einem rohrförmigen Ausgangsmaterial durch spanlose Verformung konusförmig derart verformt, dass der Innendurchmesser der Bohrung des Handstückes 1 im Bereich des Handstückes 1 a zumindest an dessen freien Ende graduell im Wesentlichen auf den Außendurchmesser der Lichtleitfaser verringert ist, so dass sich eine präzise Führung der Lichtleitfaser in dem Führungsstück 1a ergibt.

Die spanlose Verformung kann beispielsweise durch Pressen oder Schmieden durchgeführt werden.

Dadurch entstehen keine Stoßkanten, die das Ein- und Hindurchführen des freien Endes der empfindlichen Lichtleitfaser behindern oder zu Beschädigungen des Faserendes führen könnten.

Gegebenenfalls kann die Bohrung in dem Führungsstück 1a nach der spanlosen Verformung, beispielsweise durch Bohren oder Honen, nachbearbeitet werden, um eine sehr präzise Anpassung an den Außendurchmesser der Lichtleitfaser zu erzielen.

Obwohl das Führungsstück 1a in den Zeichnungen als gerades Führungsstück dargestellt ist, kann es je nach Anwendungszweck entlang seiner Längsachse abgebogen sein. Dieses Abbiegen kann vor oder nach der Verringerung seines Durchmessers erfolgen.

An dem dem Führungsstück gegenüberliegenden Ende ist das Handstück 1 mit einer Klemmvorrichtung versehen, die an der Ummantelung der Lichtleitfaser angreift, so dass keine Gefahr der Beschädigung der Lichtleitfaser selbst besteht.

Bei der in den Figuren 1 bis 4 und insbesondere in Figur 3 dargestellten Ausführungsform des Handstückes ist das dem Führungsstück 1 a gegenüberliegende Ende des Handstückes 1 mit einem Außengewinde versehen, auf das eine Überwurfmutter aufschraubbar ist, die an ihrem verschlossenen Ende eine Durchgangsbohrung für den Durchgang der ummantelten Lichtleitfaser aufweist. Zwischen diesem mit Außengewinde versehenen Ende des Handstückes 1 und dem Inneren der Überwurfmutter ist eine elastische Scheibe mit einer Durchgangsbohrung für die ummantelte Lichtleitfaser angeordnet, die beim Aufschrauben der Überwurfmutter auf das zweite Ende des Schaftes verformt und radial gegen die Ummantelung der Lichtleitfaser angepresst wird, so dass sie an dieser Stelle in Längsrichtung festgelegt wird.

Bei dieser Ausgestaltung des Handstückes kann gegebenenfalls die Überwurfmutter 2 und die elastische Scheibe 3 als Einmalartikel ausgebildet sein, die vor einem erneuten Gebrauch im Gegensatz zu dem eigentlichen Handstück 1 nicht autoklaviert wird sondern ersetzt wird.

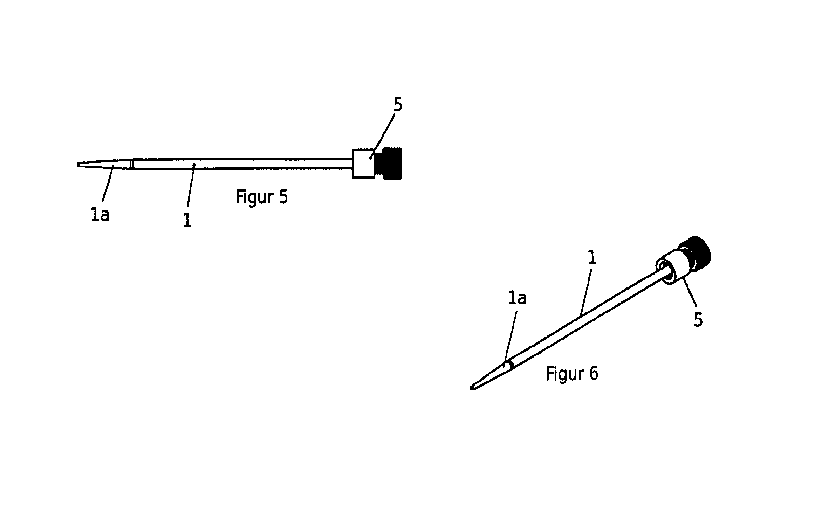

Gemäß den in den Figuren 5 und 6 gezeigten Ausführungsformen kann das dem Führungsstück 1a gegenüberliegende Ende des Handstückes 1 in einen Luer-Lock-Adapter 5 oder einen Touhy-Borst-Adapter 5 eingesetzt werden, der ein handelsübliches, in der Medizin vielfach verwendetes Bauelement darstellt.

Da das Führungsstück in jedem Falle als einstückiger Teil des Handstückes ausgebildet ist, ergibt sich eine einfache und präzise Herstellung mit geringen Kosten, und das Handstück selbst ist aufgrund seiner Herstellung aus einem geeigneten Metall sicher autoklavierbar.

Das Ausgangsmaterial des Schaftes kann insbesondere ein Edelstahlrohr sein, oder es kann ein anderes leicht verformbares Metall, wie z.B. Neusilber verwendet werden. Im letzten Fall ergibt sich der zusätzliche Vorteil, dass das Führungsstück von Hand nach Bedarf abgebogen werden kann.

## Patentansprüche

1. Verfahren zur Herstellung eines Laser-Handstücks, das zur Führung einer Lichtleitfaser mit einem ersten Außendurchmesser und mit einer die Lichtleitfaser umgebenden Ummantelung mit einem zweiten Außendurchmesser ausgebildet ist, wobei das Laser-Handstück aus einem hohlen lang gestreckten Schaft (1), einem verjüngten Führungsstück (1a), das zur Führung des von der Ummantelung befreiten Endteils der Lichtleitfaser ausgebildet ist, an einem ersten Ende des Schaftes (1) und einer Klemmvorrichtung (2) an dem dem ersten Ende entgegengesetzten zweiten Ende des Schaftes (1) besteht, wobei das Verfahren die folgenden Schritte umfasst:
einstückiges Herstellen des Schaftes (1) und des Führungsstückes aus einem mit einer Bohrung versehenen Metallrohr, dessen Innendurchmesser größer als der zweite Außendurchmesser ist,
konusförmiges spanloses Verformen eines ersten Endes des Metallrohres derart, dass der Innendurchmesser des Führungsstücks (1a) auf den ersten Außendurchmesser verringert wird, der zur präzisen Führung des Endteils der Lichtleitfaser in dem Führungsstück (1a) ausgebildet ist.

2. Verfahren nach Anspruch 1, **dadurch gekennzeichnet, dass** der Innendurchmesser des Führungsstückes (1a) am ersten Ende des Schaftes (1) exakt auf den ersten Außendurchmesser nachbearbeitet wird.

3. Laser-Handstück, das zur Führung einer Lichtleitfaser mit einem ersten Außendurchmesser und mit einer die Lichtleitfaser umgebenden Ummantelung mit einem zweiten Außendurchmesser ausgebildet ist, wobei das Laser-Handstück aus einem hohlen lang gestreckten Schaft (1), einem verjüngten Führungsstück (1 a) an einem ersten Ende des Schaftes (1) und einer Klemmvorrichtung (2) für die Ummantelung an dem dem ersten Ende entgegengesetzten zweiten Ende des Schaftes (1) besteht, wobei der Schaft (1) eine Bohrung aufweist, und
zusammen mit dem Führungsstück (1a) einstückig aus einem Metallrohr hergestellt ist, dessen Innendurchmesser größer als der zweite Außendurchmesser ist, wobei das Metallrohr zur Bildung des Führungsstücks an dem ersten Ende durch spanlose Verformung konusförmig derart verformt ist, dass der Innendurchmesser des Führungsstücks auf den ersten Außendurchmesser verringert ist.

4. Handstück nach Anspruch 3, **dadurch gekennzeichnet, dass** der Innendurchmesser des Führungsstücks lediglich an dessen freiem Ende auf den ersten Außendurchmesser verringert ist.

5. Handstück nach Anspruch 3 oder 4, **gekennzeichnet durch** eine Überwurfmutter (2), welche auf das zweite Ende des Schaftes (1) welches mit einem Außengewinde versehen ist, aufschraubbar ist eine und **dadurch** Klemmvorrichtung bildet wobei die Überwurfmutter (2) an ihrem verschlossenen Ende eine Durchgangsbohrung aufweist, die für den Durchgang der mit der außen liegenden Ummantelung versehenen Lichtleitfaser ausgebildet ist, und dass im Inneren der Überwurfmutter eine elastische Scheibe (3) mit einer Durchgangsbohrung angeordnet ist, die für die mit der Ummantelung versehene Lichtleitfaser ausgebildet und beim Aufschrauben der Überwurfmutter (2) auf das zweite Ende des Schaftes (1) verformbar und gegen die mit der Ummantelung anpressbar ist.

6. Handstück nach Anspruch 3 oder 4, **dadurch gekennzeichnet, dass** das zweite Ende des Schaftes (1) mit einem Luer-Lock-Adapter verbunden ist, durch den die Klemmung der mit der Ummantelung versehenen Lichtleitfaser erfolgt.

7. Handstück nach Anspruch 3 oder 4, **dadurch gekennzeichnet, dass** das zweite Ende des Schaftes (1) mit einem Touhy-Borst-Adapter verbunden ist, durch den die Klemmung der mit der Ummantelung versehenen Lichtleitfaser erfolgt.

8. Handstück nach einem der vorhergehenden Ansprüche, **dadurch gekennzeichnet, dass** der Innendurchmesser des Führungsstücks (1 a) an seinem freien Ende spanend nachbearbeitet ist.

9. Handstück nach einem der Ansprüche 3 bis 8, **dadurch gekennzeichnet, dass** das Führungsstück (1a) entlang seiner Längsachse abgebogen ist.

10. Handstück nach einem der Ansprüche 3 bis 9, **dadurch gekennzeichnet, dass** das Metallrohr ein Edelstahlrohr ist.

11. Handstück nach einem der Ansprüche 3 bis 9, **dadurch gekennzeichnet, dass** das Metallrohr aus leicht verformbarem Material hergestellt ist.

12. Handstück nach Anspruch 11, **dadurch gekennzeichnet, dass** das Metallrohr aus Neusilber besteht.

13. Handstück nach Anspruch 11 oder 12, **dadurch gekennzeichnet, dass** das Führungsstück (1a) von Hand abbiegbar ist.

## Claims

1. A method for the production of a laser handpiece adapted for guiding a fibre optical waveguide having a first outer diameter and a sheath of a second outer diameter, the laser handpiece comprising a hollow elongated shaft (1), a tapered guiding piece (1a) disposed at a first end of the shaft (1) and adapted for guiding the end portion of the fibre optical waveguide having the sheath removed therefrom, and a clamping device at a second end of the shaft (1) opposite to said first end, the method comprising the following steps:
integrally forming the shaft (1) and the guiding piece from a metal tube provided with a bore having an inner diameter which is greater than the second outer diameter,
non-cutting deformation of a first end of the metal tube in a cone-shaped manner such that the inner diameter of the guiding piece (1 a) is reduced to the first outer diameter adapted for precisely guiding the end portion of the fibre optical waveguide within the guiding piece (1a).

2. Method according to claim 1, **characterized in that** the inner diameter of the guiding piece (1a) at the first end of the shaft (1) is exactly finished to the first outer diameter.

3. Laser handpiece adapted for guiding a fibre optical waveguide having a first outer diameter and a sheath surrounding the fibre optical waveguide and having a second outer diameter, the laser handpiece comprising a hollow elongated shaft (1), a tapered guiding piece (1a) at a first end of the shaft (1) and a clamping device (2) for the sheath at the second end of the shaft (1) opposite to the first end, the shaft (1) having a bore, and is formed together with the guiding piece (1a) in one piece from a metal tube having an inner diameter greater than the second outer diameter, the metal tube being deformed for forming the guiding piece by means of none-cutting deformation in a cone-shaped manner such that the inner diameter of the guiding piece is reduced to the first outer diameter.

4. Handpiece as claimed in claim 3, **characterized in that** the inner diameter of the guiding piece is reduced only at the free end thereof to the first outer diameter.

5. Handpiece as claimed in claim 3 or 4, **characterized by** an union nut (2) adapted to be screwed onto the second end of the shaft (1) provided with an external thread, said union nut forming a clamping device having at its closed end a through bore which is adapted for passing the fibre optical waveguide provided with the outer sheath, and wherein within the union nut an elastic disk (3) having a through bore adapted for the fibre optical waveguide provided with the sheath is disposed said disk being deformable and compressable against the sheath on screwing the union nut (2) onto the second end of the shaft (1).

6. Handpiece according to claim 3 or 4, **characterized in that** the second end of the shaft (1) is provided with a Luer-Lock-Adapter by which the clamping of the fibre optical waveguide provided with the sheath is effected.

7. Handpiece according to claim 3 or 4, **characterized in that** the second end of the shaft (1) is connected to a Touhy-Borst-Adapter by which the clamping of the fibre optical waveguide provided with the sheath is effected.

8. Handpiece according to any of the preceding claims, **characterized in that** the inner diameter of the guiding piece (1 a) is re-finished at its free end by metal cutting.

9. Handpiece according to any of the claims 3 through 8, **characterized in that** the guiding piece (1a) is bent along its longitudinal axis.

10. Handpiece according to any of the claims 3 through 9, **characterized in that** the metal tube is a stainless steel tube.

11. Handpiece according to any of the claim 3 through 9, **characterized in that** the metal tube is formed from readily deformable material.

12. Handpiece according to claim 11, **characterized in that** the metal tube consists of alpaka.

13. Handpiece according to claim 11 or 12, **characterized in that** the guiding piece (1a) is adapted to be bent by hand.

## Revendications

1. Procédé pour la fabrication d'une pièce à main laser, laquelle est réalisée pour le guidage d'une fibre optique avec un premier diamètre extérieur et avec une gaine entourant la fibre optique avec un deuxième diamètre extérieur, moyennant quoi la pièce à main laser est constituée d'une tige (1) creuse allongée, d'une pièce de guidage effilée (la), laquelle est réalisée pour le guidage de la partie d'extrémité libérée de la gaine de la fibre optique, à une première extrémité de la tige (1), et d'un dispositif de serrage (2) à la seconde extrémité de la tige (1) opposée à la première extrémité, le procédé comprenant les étapes suivantes :
fabrication d'une seule pièce de la tige (1) et de la pièce de guidage à partir d'un tube métallique muni d'un alésage dont le diamètre intérieur est supérieur au deuxième diamètre extérieur,
formage en forme de cône sans enlèvement de copeaux d'une première extrémité du tube métallique de manière à ce que le diamètre intérieur de la pièce de guidage (1a) soit réduit au premier diamètre extérieur, lequel est réalisé pour le guidage précis de la partie d'extrémité de la fibre optique dans la pièce de guidage (1a).

2. Procédé selon la revendication 1, **caractérisé en ce que** le diamètre intérieur de la pièce de guidage (1a) à la première extrémité de la tige (1) est rectifié de manière exacte afin de correspondre au premier diamètre extérieur.

3. Pièce à main laser, laquelle est réalisée pour le guidage d'une fibre optique avec un premier diamètre extérieur et avec une gaine enveloppant la fibre optique avec un deuxième diamètre extérieur, moyennant quoi la pièce à main laser est constituée d'une tige (1) creuse allongée, d'une pièce de guidage effilée (1a) à une première extrémité de la tige (1) et d'un dispositif de serrage (2) pour la gaine au niveau de la deuxième extrémité de la tige (1) opposée à la première extrémité, moyennant quoi la tige (1) présente un alésage et est fabriquée d'une seule pièce ensemble avec la pièce de guidage (1a) à partir d'un tube métallique dont le diamètre intérieur est plus grand que le deuxième diamètre extérieur, moyennant quoi le tube métallique est soumis à un formage en forme de cône sans enlèvement de copeaux pour la formation de la pièce de guidage à la première extrémité de manière à ce que le diamètre intérieur de la pièce de guidage soit réduit au premier diamètre extérieur.

4. Pièce à main selon la revendication 3, **caractérisée en ce que** le diamètre intérieur de la pièce de guidage est réduit au premier diamètre extérieur seulement au niveau de l'extrémité libre de celle-ci.

5. Pièce à main selon les revendication 3 ou 4, **caractérisée par** un écrou de raccord (2) qui peut être vissé sur la deuxième extrémité de la tige (1), laquelle est munie d'un filetage extérieur et forme un dispositif de serrage, moyennant quoi l'écrou de raccord (2) présente un alésage traversant sur son extrémité fermée, lequel est réalisé pour le passage de la fibre optique munie de la gaine située à l'extérieur, et en ce qu'à l'intérieur de l'écrou de raccord est disposé un disque élastique (3) avec un alésage traversant, lequel est réalisé pour la fibre optique munie de la gaine et peut se déformer lors du vissage de l'écrou raccord (2) sur la deuxième extrémité de la tige (1) et peut être pressé contre la gaine.

6. Pièce à main selon les revendications 3 ou 4, **caractérisée en ce que** la deuxième extrémité de la tige (1) est reliée à un adaptateur Luer-Lock grâce auquel s'effectue le serrage de la fibre optique munie de l'enveloppe.

7. Pièce à main selon les revendications 3 ou 4, **caractérisée en ce que** la deuxième extrémité de la tige (1) est reliée à un adaptateur Touhy-Borst, grâce auquel s'effectue le serrage de la fibre optique munie de la gaine.

8. Pièce à main selon l'une des revendications précédentes, **caractérisée en ce que** le diamètre intérieur de la pièce de guidage (1a) est rectifié par enlèvement de copeaux sur son extrémité libre.

9. Pièce à main selon l'une des revendications 3 à 8, **caractérisée en ce que** la pièce de guidage (1a) est recourbée le long de son axe longitudinal.

10. Pièce à main selon l'une des revendications 3 à 9, **caractérisée en ce que** le tube métallique est un tube en acier noble.

11. Pièce à main selon l'une des revendications 3 à 9, **caractérisée en ce que** le tube métallique est fabriqué en un matériau facilement déformable.

12. Pièce à main selon la revendication 11, **caractérisée en ce que** le tube métallique se compose d'argentan.

13. Pièce à main selon les revendications 11 ou 12, **caractérisée en ce que** la pièce de guidage (1a) peut être recourbée manuellement.
